# EUROPÄISCHE PATENTANMELDUNG

(11) **EP 1 600 126 A1**
(43) Veröffentlichungstag der Anmeldung: **30.11.2005**
(21) Anmeldenummer: 04012774.8
(22) Anmeldetag: 28.05.2004
(51) Int. Cl.: A61F 2/20

(54) **Zungenbeinprothese**

(71) Anmelder: Herrmann, Ingo F., Prof. Dr., 81479 München (DE)
(72) Erfinder: Herrmann, Ingo F., Prof. Dr., 81479 München (DE)
(74) Vertreter: Manitz, Finsterwald & Partner GbR

(57) **Zusammenfassung**

Eine Zungenbeinprothese besitzt wenigstens einen Befestigungsabschnitt (11). Dieser gestattet die Befestigung eines Verbindungsbands (27). Somit kann nach einer Amputation von Kehlkopf und Zungenbein die Überzungenbeinmuskulatur mit dem oberen Ende der Luftröhre verbunden werden, um die Atem-, Stimm- und Schluckfunktion wiederherzustellen.

## Beschreibung

Die Erfindung betrifft eine Zungenbeinprothese. Hintergrund der Erfindung ist die bei fortgeschrittenem Tumorbefall erforderliche Amputation des Kehlkopfes (Larynx). In der weit überwiegenden Anzahl der Fälle wird zusammen mit dem Kehlkopf auch das Zungenbein (Os hyoideum) entfernt. Sofern ein Kehlkopfkarzinom frühzeitig erkannt und der Kehlkopf (gegebenenfalls mit Zungenbein) entsprechend früh entfernt wird, besteht generell eine günstige Genesungsprognose. Allerdings sind nach erfolgter Kehlkopfamputation die Atem-, Stimm- und Schluckfunktionen des betroffenen Patienten erheblich eingeschränkt. Die mit totaler Kehlkopfamputation einhergehende Luftröhrenöffnung im Hals (Tracheostoma) bedeutet für den Patienten eine dauerhafte psychische Belastung und erschwert den gesellschaftlichen Umgang.

Eine Aufgabe der Erfindung besteht darin, die vorgenannten Probleme zu beseitigen oder zumindest zu lindern, und eine weitgehende Rekonstruktion des amputierten Kehlkopfes zu ermöglichen.

Diese Aufgabe wird durch eine Zungenbeinprothese gelöst, die einem Patienten nach Amputation von Kehlkopf und Zungenbein implantiert werden kann und die wenigstens einen Befestigungsabschnitt aufweist, an dem wenigstens ein zur Luftröhre führendes Verbindungsband befestigbar ist.

Eine derartige Zungenbeinprothese kann einerseits mit der Überzungenbeinmuskulatur (suprahyoidale Muskulatur) fest verbunden werden. Andererseits ermöglicht es die Prothese, mittels eines Verbindungsbands eine Verbindung zum oberen Ende der Luftröhre herzustellen. Letztlich wird mittels der erfindungsgemäßen Prothese also die Überzungenbeinmuskulatur mit der Luftröhre verbunden. Auf diese Weise kann das künstliche Zungenbein die zentrale Steuerungsfunktion für eine Rekonstruktion des amputierten Kehlkopfes übernehmen. Somit wird nach einer Kehlkopfamputation eine Wiederherstellung der Atem- und Schluckfunktionen sowie in einigen Fällen der Stimmfunktion möglich. Ein Tracheostoma und die hiermit verbundenen Probleme können vermieden werden.

Kennzeichnend für die erfindungsgemäße Zungenbeinprothese ist die Ausgestaltung mit einem oder mehreren Befestigungsabschnitten. Über die Befestigungsabschnitte kann an der Prothese ein zur Luftröhre führendes Verbindungsband befestigt werden. Somit kann die erläuterte Verbindung zwischen Überzungenbeinmuskulatur und Luftröhre in zwei Schritten hergestellt werden: Erst wird die Zungenbeinprothese eingesetzt und mit der Überzungenbeinmuskulatur vernäht. Nach einem Einheilungsprozess wird die Zungenbeinprothese in einem zweiten Schritt mit der Luftröhre verbunden. Zu diesem Zweck wird das Verbindungsband nachträglich an dem oder den Befestigungsabschnitten der bereits implantierten Prothese befestigt und mit der Luftröhre verbunden.

Bei dem Verbindungsband handelt es sich vorzugsweise um eine Sehne oder einen Sehnenabschnitt, die bzw. der dem Patienten beispielsweise aus dem Beinbereich entnommen wird. Dadurch sind eine stabile Verbindung und eine gute Körperverträglichkeit gewährleistet. Alternativ hierzu kann ein künstliches Verbindungsband vorgesehen sein.

Vorzugsweise sind mehrere Befestigungsabschnitte vorgesehen. Hierdurch kann eine besonders gleichmäßige Kräfteverteilung bewirkt werden. Beispielsweise können zwei bis sechs oder mehr Befestigungsabschnitte vorgesehen sein, an denen ein einziges Verbindungsband befestigt ist, das zur Luftröhre führt. Die Zungenbeinprothese kann beispielsweise auch mit vier Befestigungsabschnitten versehen sein, an denen jeweils paarweise zwei Verbindungsbänder befestigt sind.

Es ist ferner bevorzugt, wenn die Befestigungsabschnitte in einer symmetrischen Anordnung bezüglich der Mittensymmetrieebene der Zungenbeinprothese vorgesehen sind. Beispielsweise können zwei, vier oder sechs Befestigungsabschnitte paarweise symmetrisch zueinander angeordnet sein. Drei oder fünf Befestigungsabschnitte können beispielsweise an den Eckpunkten eines gleichschenkligen Dreiecks oder eines spiegelsymmetrischen Fünfecks angeordnet sein, wobei ein mittlerer Befestigungsabschnitt auf der Mittensymmetrieebene der Zungenbeinprothese liegt. Derartige symmetrische Anordnungen bewirken eine gleichmäßige Verteilung der Zugkräfte zwischen der Überzungenbeinmuskulatur und der Luftröhre.

Der Befestigungsabschnitt oder die Befestigungsabschnitte können an der Unterseite, oder an wenigstens einer Seitenfläche, oder sowohl an der Unterseite als auch an wenigstens einer Seitenfläche, oder am Übergang zwischen der Unterseite und wenigstens einer Seitenfläche der Zungenbeinprothese angeordnet sein. In diesen Fällen kann das an der Zungenbeinprothese befestigte Verbindungsband ergonomisch günstig in Richtung der Luftröhre verlaufen.

Vorzugsweise sind der Befestigungsabschnitt oder die Befestigungsabschnitte der Prothese durch jeweils eine Befestigungsöse gebildet, die zur Aufnahme oder zum Durchziehen des Verbindungsbands ausgebildet ist. Die Befestigungsöse oder die Befestigungsösen besitzen vorzugsweise eine längliche Öffnung und eine glatte Auflagefläche für eine gleichmäßige Verteilung der an dem Verbindungsband wirkenden Zugkräfte.

In diesem Fall ist es bevorzugt, wenn die Befestigungsöse oder die Befestigungsösen der Zungenbeinprothese zur Aufnahme einer Verbindungsschlinge geeignet sind, d.h. eines umfänglich geschlossenen Verbindungsbands, das durch die Befestigungsösen geführt ist. Eine derartige Verbindungsschlinge kann besonders gut um das obere Ende der Luftröhre geführt bzw. mit der Luftröhre verbunden werden. Eine Verbindungsschlinge, die beweglich durch eine oder mehrere Befestigungsösen geführt ist, begünstigt ferner eine symmetrische Kräfteverteilung seitens der Zungenbeinprothese. Die Befestigungsöse oder die Befestigungsösen sind besonders geeignet für eine Verbindungsschlinge, wenn sie mit abgerundeten Umlenkabschnitten versehen sind, die den betreffenden Abschnitt der Verbindungsschlinge in schonender Weise in Richtung der Luftröhre oder in Richtung einer weiteren Befestigungsöse umlenken.

Eine derartige Verbindungsschlinge kann besonders gut aus einer Sehne des Patienten gebildet werden, nämlich indem die beiden Enden der Sehne über eine oder mehrere Längsnähte miteinander verbunden werden.

Alternativ zu der Befestigung über die genannten Befestigungsösen kann das Verbindungsband an dem oder den Befestigungsabschnitten beispielsweise vernäht werden. In diesem Fall kann der Befestigungsabschnitt zum Beispiel als Zungenfortsatz der Prothese ausgebildet sein, der mit einer Nähnadel besonders gut durchdrungen werden kann.

Ferner ist es bevorzugt, wenn die Zungenbeinprothese mehrere Nahtösen besitzt, die ein Annähen der Zungenbeinprothese an die Überzungenbeinmuskulatur ermöglichen. Mit Hilfe der Nahtösen kann also nach dem Einsetzen der Zungenbeinprothese die bereits erläuterte Verbindung zu der Überzungenbeinmuskulatur hergestellt werden. Zu diesem Zweck können beispielsweise sechs bis zwölf Nahtösen vorgesehen sein, insbesondere in einer gleichmäßigen und/oder symmetrischen Anordnung. Auch die Nahtösen sind vorzugsweise an der Unterseite, oder an wenigstens einer Seitenfläche, oder sowohl an der Unterseite als auch an wenigstens einer Seitenfläche, oder am Übergang zwischen der Unterseite und wenigstens einer Seitenfläche der Zungenbeinprothese angeordnet.

Die Zungenbeinprothese besteht im Wesentlichen aus einem Material, das sich im Gewebe inert verhält, also nicht zur Narbenbildung oder zu Fibrosierungen führt. Beispielsweise kommt ein fester Kunststoff oder Titan in Betracht.

An der Oberseite ist die Zungenbeinprothese vorzugsweise mit einer Schicht aus einem Material versehen, das die Einheilung in die Überzungenbeinmuskulatur begünstigt. Beispielsweise kann die Prothese mit Polytetrafluorethylen (PTFE) belegt oder beschichtet sein, das auch unter dem Handelsnamen Teflon (eingetragene Marke) bekannt ist.

Weiterhin ist es bevorzugt, wenn die Zungenbeinprothese eine Bogenform besitzt, d.h. im Wesentlichen C- oder U-förmig ist.

Gemäß einer vorteilhaften Weiterbildung kann die Zungenbeinprothese inelastisch verformbar sein, so dass sie beim Einsetzen in den Patienten auf einfache Weise an die konkreten anatomischen Gegebenheiten angepasst werden kann und diese Form nach dem Einsetzen auch beibehält.

Schließlich bezieht sich die Erfindung auch auf ein Verfahren zur Implantation einer Zungenbeinprothese, bei dem die folgenden Schritte durchgeführt werden:
- Vernähen der Zungenbeinprothese an der Überzungenbeinmuskulatur; und
- Verbinden der Zungenbeinprothese mit einem Luftröhrenabschnitt mittels eines Verbindungsbands, insbesondere mittels einer Verbindungsschlinge.

Zwischen dem Schritt des Vernähens der Prothese an der Überzungenbeinmuskulatur und dem Schritt des Verbindens der Prothese mit dem Luftröhrenabschnitt ist vorzugsweise ein Einheilprozess vorgesehen, d.h. das Implantationsverfahren wird in zwei separaten chirurgischen Eingriffen vorgenommen.

Für den Schritt des Verbindens der Prothese mit dem Luftröhrenabschnitt wird vorzugsweise wenigstens ein Verbindungsband an der Prothese befestigt, das auch mit der Luftröhre verbunden wird.

Vor dem Schritt des Verbindens der Prothese mit dem Luftröhrenabschnitt kann dem Patienten eine Sehne entnommen werden, die als Verbindungsband oder Verbindungsschlinge dient.

Weitere Ausführungsformen der Erfindung sind in den Unteransprüchen beschrieben.

Die Erfindung wird nachfolgend lediglich beispielhaft unter Bezugnahme auf die Zeichnungen erläutert.
- Fig. 1: zeigt eine Perspektivansicht einer erfindungsgemäßen Zungenbeinprothese von seitlich schräg oben.
- Fig. 2: zeigt eine Unteransicht der Prothese.
- Fig. 3: zeigt eine Frontansicht der Prothese.
- Fig. 4: zeigt eine Draufsicht der Prothese.
- Fig. 5: zeigt eine Seitenansicht der Zungenbeinprothese gemäß Fig. 1 bis 4, wobei die Prothese mit der Überzungenbeinmuskulatur vernäht und mit einer Sehnenschlinge verbunden ist.

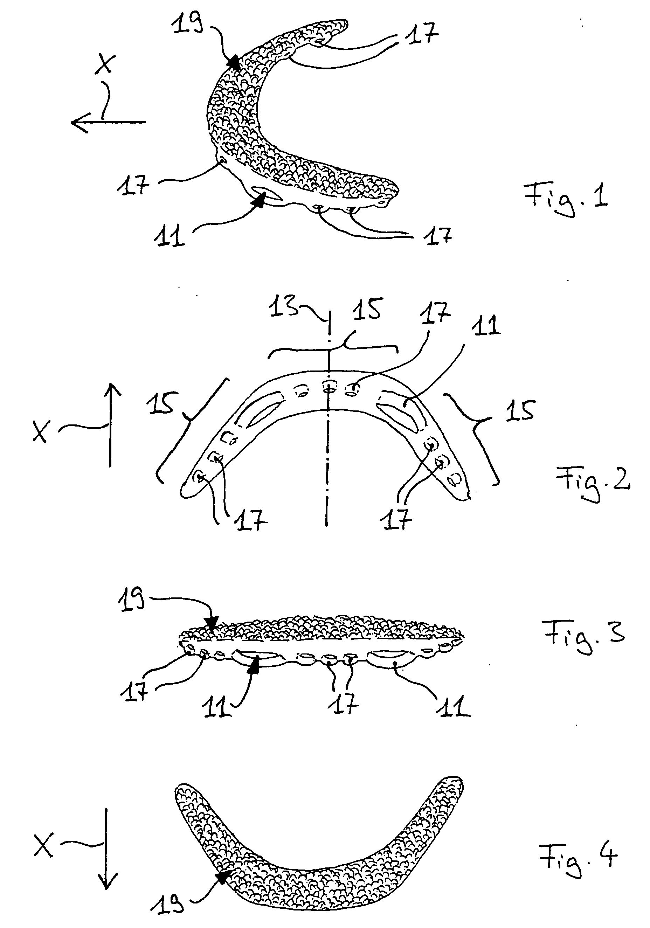

Das in den Fig. 1 bis 5 gezeigte künstliche Zungenbein besitzt einen im Wesentlichen bogenförmigen oder hufeisenförmigen Grundkörper aus Kunststoff oder Titan.

Wie aus Fig. 2 ersichtlich ist, besitzt die Prothese an der Unterseite zwei Befestigungsösen 11, die als Befestigungsabschnitte zur Aufnahme einer Verbindungsschlinge dienen, wie nachfolgend noch erläutert wird. Die Befestigungsösen 11 sind bezüglich einer Mittensymmetrieebene 13 der Prothese spiegelsymmetrisch angeordnet. Die beiden Befestigungsösen 11 unterteilen die Bogenform der Prothese in drei etwa gleich lange Längsabschnitte 15. Entlang jedes Längsabschnitts 15 sind an der Unterseite der Prothese drei Nahtösen 17 ausgebildet, d.h. jede Befestigungsöse 11 ist beidseitig von jeweils drei Nahtösen 17 umgeben.

Die Befestigungsösen 11 sind deutlich größer als die Nahtösen, und sie sind ausreichend groß, um ein Verbindungsband oder eine Sehne durch sie durchführen bzw. durchziehen zu können. Beispielsweise können die Befestigungsösen 11 eine Breite von 2 bis 6 mm und eine Höhe bis 2 mm besitzen. Die Nahtösen 17 dienen demgegenüber lediglich zum Durchziehen eines Nähfadens (z.B. Ethibond 2-0). Die jeweilige Durchlassrichtung der Befestigungsösen 11 und der Nahtösen 17 verläuft ungefähr senkrecht zu dem jeweiligen Längsabschnitt 15 und somit etwa parallel zu dem Krümmungsradius der Bogenform der Prothese.

Wie insbesondere aus den Fig. 1, 3 und 4 ersichtlich ist, besitzt die gezeigte Zungenbeinprothese an der Oberseite eine PTFE-Schicht 19, die nach oben gerichtete kleine Schlingen und Faserstücke aus Polytetrafluorethylen aufweist.

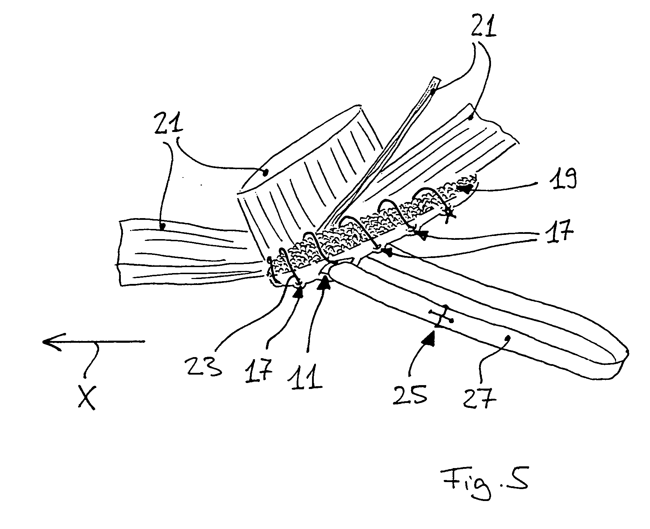

Die gezeigte Zungenbeinprothese dient als Ersatz für das natürliche Zungenbein, nachdem dieses zusammen mit dem Kehlkopf aus Tumorgründen amputiert worden ist. Nach Implantation ist die Zungenbeinprothese mit der Überzungenbeinmuskulatur 21 verbunden (vgl. Fig. 5). Ferner wird die Zungenbeinprothese mittels der Befestigungsösen 11 und eines hieran befestigten Verbindungsbands oder einer hieran befestigten Verbindungsschlinge mit der Luftröhre verbunden. Hierdurch können Bewegungen der Überzungenbeinmuskulatur auf die Luftröhre übertragen werden. Der amputierte Kehlkopf kann somit weitgehend rekonstruiert werden, und die nach einer Kehlkopfamputation üblicherweise auftretenden Atem-, Stimm- und Schluckprobleme werden erheblich gelindert.

Im Einzelnen kann die Implantation der gezeigten Zungenbeinprothese wie folgt geschehen:

Nach Amputation des Kehlkopfes und des natürlichen Zungenbeins wird die gezeigte Zungenbeinprothese unter die Überzungenbeinmuskulatur 21 des Patienten eingesetzt und mit dieser vernäht, wie in der Seitenansicht gemäß Fig. 5 dargestellt ist. Zu diesem Zweck wird ein Nähfaden 23 durch die Nahtösen 17 und durch die Überzungenbeinmuskulatur 21 geführt. Vorzugsweise wird hierbei eine einzige Naht gesetzt, so dass lediglich zwei Knoten erforderlich sind, nämlich an den beiden Enden der Prothese.

Hierdurch wird eine gleichmäßigere Kräfteverteilung entlang der Naht erreicht. Es ist jedoch auch möglich, für jede Nahtöse 17 eine eigene Naht vorzusehen. In beiden Fällen kann der Nähfaden 23 zusätzlich auch durch die Befestigungsösen 11 geführt werden.

Danach folgt ein Einheilungsprozess von einigen Tagen bis Wochen, wobei die Zungenbeinprothese über die PTFE-Schicht 19 und die daran ausgebildeten Schlingen und Fasern eine feste und dauerhafte Verbindung mit der Überzungenbeinmuskulatur 21 eingeht.

Nach Beendigung des Einheilungsprozesses wird die Zungenbeinprothese mit der Luftröhre des Patienten dergestalt verbunden, dass Zugkräfte übertragen werden können. Zu diesem Zweck wird wenigstens ein Verbindungsband mit der Zungenbeinprothese einerseits und mit der Luftröhre andererseits verbunden. Insbesondere wird dem Patienten an anderer Stelle ein Sehnenband entnommen, das durch die beiden Befestigungsösen 11 gezogen, um die Luftröhre herum geführt (in Fig. 5 nicht gezeigt), und schließlich an einer Verbindungsstelle 25 zu einer geschlossenen Sehnenschlinge 27 vernäht wird. Das benötigte Sehnenband kann dem Patienten beispielsweise aus dem Beinbereich entnommen werden.

Nach diesem zweiten Eingriff übernimmt die Zungenbeinprothese eine Steuerungsfunktion für die Luftröhre des Patienten. Insbesondere kann der Patient mittels der Überzungenbeinmuskulatur 21, der hiermit verbundenen Zungenbeinprothese und der hieran befestigten Sehnenschlinge 27 eine Bewegung der Luftröhre entlang einer Vorwärtsrichtung X auslösen. Die Überzungenbeinmuskulatur 21 wiederum ist entlang der Vorwärtsrichtung X letztlich mit dem Kinnbereich, entgegen der Vorwärtsrichtung X mit den Ohrbereichen und nach oben mit dem Unterkiefer des Patienten verbunden.

### Bezugszeichenliste

- 11: Befestigungsöse
- 13: Mittensymmetrieebene
- 15: Längsabschnitt
- 17: Nahtöse
- 19: PTFE-Schicht
- 21: Überzungenbeinmuskulatur
- 23: Nähfaden
- 25: Verbindungsstelle
- 27: Sehnenschlinge

- X: Vorwärtsrichtung

## Patentansprüche

1. Zungenbeinprothese, **gekennzeichnet durch** wenigstens einen Befestigungsabschnitt (11), an dem wenigstens ein zur Luftröhre führendes Verbindungsband (27) befestigbar ist.

2. Zungenbeinprothese nach Anspruch 1,
**dadurch gekennzeichnet,**
**dass** zwei bis sechs Befestigungsabschnitte (11) vorgesehen sind, wobei die Befestigungsabschnitte (11) insbesondere symmetrisch bezüglich der Mittensymmetrieebene (13) der Zungenbeinprothese angeordnet sind.

3. Zungenbeinprothese nach einem der vorhergehenden Ansprüche,
**dadurch gekennzeichnet,**
**dass** der Befestigungsabschnitt (11) zur Befestigung eines Verbindungsbands (27) geeignet ist, das durch eine Sehne oder aus Kunststoff gebildet ist.

4. Zungenbeinprothese nach einem der vorhergehenden Ansprüche,
**dadurch gekennzeichnet,**
**dass** der Befestigungsabschnitt (11) an der Unterseite und/oder an wenigstens einer Seitenfläche der Zungenbeinprothese vorgesehen ist.

5. Zungenbeinprothese nach einem der vorhergehenden Ansprüche,
**dadurch gekennzeichnet,**
**dass** der Befestigungsabschnitt eine Befestigungsöse (11) aufweist.

6. Zungenbeinprothese nach Anspruch 5,
**dadurch gekennzeichnet,**
**dass** die Befestigungsöse (11) zur Aufnahme einer Verbindungsschlinge geeignet ist,
und/ oder
**dass** die Befestigungsöse (11) eine Breite von 2 bis 6 mm und eine Höhe bis 2 mm besitzt.

7. Zungenbeinprothese nach einem der vorhergehenden Ansprüche,
**dadurch gekennzeichnet,**
**dass** die Zungenbeinprothese mehrere Nahtösen (17) zum Annähen der Zungenbeinprothese an die Überzungenbeinmuskulatur aufweist,
wobei die Nahtösen (17) insbesondere an der Unterseite und/oder an wenigstens einer Seitenfläche der Zungenbeinprothese vorgesehen sind.

8. Zungenbeinprothese nach Anspruch 7 in Verbindung mit Anspruch 5 oder 6,
**dadurch gekennzeichnet,**
**dass** die Befestigungsösen (11) größer sind als die Nahtösen (17).

9. Zungenbeinprothese nach einem der vorhergehenden Ansprüche,
**dadurch gekennzeichnet,**
**dass** die Zungenbeinprothese an der Oberseite mit einer Schicht aus Polytetrafluorethylen (19) versehen ist.

10. Zungenbeinprothese nach einem der vorhergehenden Ansprüche,
**dadurch gekennzeichnet,**
**dass** die Zungenbeinprothese eine Bogenform besitzt,
und/oder
**dass** die Zungenbeinprothese aus Kunststoff oder Titan besteht,
und/oder
**dass** die Zungenbeinprothese inelastisch verformbar ist.
